# EUROPÄISCHE PATENTANMELDUNG

(11) **EP 2 821 764 A1**
(43) Veröffentlichungstag der Anmeldung: **07.01.2015**
(21) Anmeldenummer: 13174475.7
(22) Anmeldetag: 01.07.2013
(51) Int. Cl.: G01K 1/12, C21C 5/46, G01K 15/00, G01N 33/20, G01R 31/28

(54) **Testgerät für eine Messlanze zur Durchführung von Messungen bei der Erzeugung und Verarbeitung von Flüssigmetallschmelzen**

(71) Anmelder: Siemens VAI Metals Technologies GmbH, 4031 Linz (AT)
(72) Erfinder: Hartl, Franz, 4720 Kallham (AT); Kuehas, Thomas, 4222 Luftenberg (AT); Lehofer, Martin, 4050 Traun (AT); Riese, Axel, 4020 Linz (AT); Rohrhofer, Andreas, 4020 Linz (AT); Weinzinger, Michael, 4020 Linz (AT)
(74) Vertreter: Maier, Daniel Oliver

(57) **Zusammenfassung**

Die Erfindung betrifft ein Testgerät (1) für eine Messlanze (10) zur Durchführung von Messungen bei der Erzeugung und Verarbeitung von Flüssigmetallschmelzen, wobei die Messlanze (10) ein Kontaktstück (11) und eine mechanisch lösbar am Kontaktstück (11) befestigbare Tauchsonde (12) aufweist, wobei die Tauchsonde (12) in die Schmelze eintauchbar ist und zumindest einen Sensor (13) zur Ausgabe eines für eine jeweilige Eigenschaft der Schmelze charakteristischen Sensorsignals aufweist, wobei das Kontaktstück (11) zumindest eine Kontaktschnittstelle (14) aufweist, mittels welcher der zumindest eine Sensor (13) mit dem Kontaktstück (11) elektrisch verbindbar ist. Weiterhin betrifft die Erfindung ein Testsystem mit einem derartigen Testgerät (1) und einer Steuereinheit (20) sowie ein Verfahren zum Betrieb eines derartigen Testsystems. Um einen verbesserten Test bezüglich der Funktion eines Kontaktstücks (11) einer Messlanze (10) bereitzustellen, wird vorgeschlagen, dass das Testgerät (1) ein Adapterstück (2) mit einer Öffnung (3), in welche das Kontaktstück (2) zumindest teilweise einführbar ist, und zumindest eine Testschnittstelle (4) aufweist, welche derart angeordnet ist, dass die zumindest eine Kontaktschnittstelle (14) des zumindest teilweise in die Öffnung (3) eingeführten Kontaktstücks (11) mit der zumindest einen Testschnittstelle (4) elektrisch verbindbar ist.

## Beschreibung

Die Erfindung betrifft ein Testgerät für eine Messlanze zur Durchführung von Messungen bei der Erzeugung und Verarbeitung von Flüssigmetallschmelzen, wobei die Messlanze ein Kontaktstück und eine mechanisch lösbar am Kontaktstück befestigbare Tauchsonde aufweist, wobei die Tauchsonde in die Schmelze eintauchbar ist und zumindest einen Sensor zur Ausgabe eines für eine jeweilige Eigenschaft der Schmelze charakteristischen Sensorsignals aufweist, wobei das Kontaktstück zumindest eine Kontaktschnittstelle aufweist, mittels welcher der zumindest eine Sensor mit dem Kontaktstück elektrisch verbindbar ist.

Weiterhin betrifft die Erfindung ein Testsystem für eine Messlanze zur Durchführung von Messungen bei der Erzeugung und Verarbeitung von Flüssigmetallschmelzen, wobei die Messlanze ein Kontaktstück und eine mechanisch lösbar am Kontaktstück befestigbare Tauchsonde aufweist, wobei die Tauchsonde in die Schmelze eintauchbar ist und zumindest einen Sensor zur Ausgabe eines für eine jeweilige Eigenschaft der Schmelze charakteristischen Sensorsignals aufweist, wobei das Kontaktstück zumindest eine Kontaktschnittstelle aufweist, mittels welcher der zumindest eine Sensor mit dem Kontaktstück elektrisch verbindbar ist.

Schließlich betrifft die Erfindung ein Verfahren zum Betrieb eines derartigen Testsystems.

Ein derartiges Testgerät und Testsystem kommen beispielsweise bei der Erzeugung von Flüssigmetallschmelzen, wie zum Beispiel in einem Stahlwerk, zum Einsatz. Zur Überwachung des Produktionsprozesses werden dabei regelmäßig Messungen durchgeführt, z. B. der Temperatur oder des Sauerstoff- und Kohlenstoffgehaltes der flüssigen Schmelze. Diese Messungen werden je Schmelze mindestens ein Mal durchgeführt. Die Ergebnisse der Messungen fließen direkt in die Steuereinheit des metallurgischen Prozesses ein. Weiters werden die Ergebnisse in Qualitätsaufzeichnungen festgehalten.

Eine Messung läuft dabei beispielsweise wie folgt ab. Eine Einwegtauchsonde mit integrierter Sensorik wird mittels eines Kontaktstücks, welches eine Schnittstelle zur mechanischen und elektrischen Kopplung darstellt, auf eine Vorrichtung an einem Manipulator, das heißt einer Lanze, aufgesteckt. Die Leitungen der Sensoren werden über Steckkontakte durch den Manipulator bis zum Mess- bzw. Analysegerät geführt. Der Manipulator bewegt automatisch die Einwegtauchsonde in die Flüssigmetallschmelze, worauf die Messungen durchgeführt werden.

Alternativ ist vorstellbar, dass die Lanze mit der Einwegtauchsonde durch einen Bediener manuell in die Flüssigmetallschmelze eingeführt wird anstatt dem Manipulator.

Bei diesem Vorgang wird die hauptsächlich aus Karton bestehende Einwegtauchsonde verzehrt, wodurch sich Verbrennungsrückstände am Kontaktstück ablagern können. Diese Verschmutzungen führen meist zu einem erhöhten elektrischen Übergangswiderstand im Bereich des Kontaktstückes, wodurch die Thermospannung verfälscht wird. Weiterhin wird das Kabelisoliermaterial abgenutzt, was zu einer Verschlechterung der Isolationswerte und in weiterer Folge zu Fehlmessungen führen kann.

Die Einwegtauchsonden werden nach der Durchführung der Messung entsorgt. Verschleiß und Verschmutzung können dazu führen, dass Messergebnisse verfälscht werden, wobei verfälschte Messergebnisse weiterreichende Folgen haben können, wie z. B.
- falsche Prozessführung aufgrund falscher Ist-Prozessgrößen,
- Verzögerungen in der Produktion durch Entdeckung des Fehlers erst im nächsten Produktionsschritt,
- Einsatz von nicht notwendigen Zuschlagstoffen,
- thermischer Entgleisung des Konverterprozesses und
- Änderung des Produktionsplans durch zeitliche Verzögerungen, z.B. erneute Messung, Zugabe von Zuschlagstoffen, ...

Im Fall einer falschen Temperaturmessung oder thermischer Entgleisung kann es zu einer unbemerkten Überhitzung und Zerstörung des Konvertergefäßes selbst kommen, was ein erhebliches Sicherheitsrisiko darstellt und hohe Kosten verursacht.

In einem geeigneten Prozesszustand wie etwa einer Unterbrechung des Blasebetriebs beim LD-Konverter begibt sich ein Instandhalter mit einem Handprüfgerät in die Gefahrenzone des Aggregates, z.B. des LD Konverters oder dergleichen, und führt folgende Tätigkeiten durch:
- Anbringen des Handgeräts am Kontaktstück,
- Start eines Simulationsprogrammes mit einer fix voreingestellten Temperatur bzw. Thermospannung,
- Kontaktaufnahme mit dem Bedienpersonal im Leitstand und Abfrage der dort angezeigten Messwerte,
- Vergleich zwischen Messwerten am Handbediengerät und im Leitstand,
- Reinigung des Kontaktstückes im Fall einer Messwertabweichung und
- neuerliche Überprüfung.

Wenn die Messwerte nicht plausibel erscheinen bzw. keine Lösung herbeigeführt werden kann, erfolgt ein Austausch des Kontaktstückes oder von weiteren Komponenten durch den Instandhalter vor Ort. Dabei kann sich die Aufenthaltsdauer des Instandhalters im Gefahrenbereich des Aggregates, also im Umfeld von CO-Gas, Hitze, Stahl- und Schlackenspritzern, zwischen 5 und 30 Minuten bewegen. In diesem Zeitraum ist er einem erhöhten Sicherheitsrisiko ausgesetzt. Aufgrund dieses Sicherheitsrisikos wird die manuelle Überprüfung zumeist nur einmal pro Tag oder seltener durchgeführt.

Der Erfindung liegt die Aufgabe zugrunde, einen verbesserten Testvorgang bezüglich der Funktion eines Kontaktstücks einer Messlanze bereitzustellen.

Diese Aufgabe wird durch ein Testgerät der eingangs genannten Art dadurch gelöst, dass das Testgerät ein Adapterstück mit einer Öffnung, in welche das Kontaktstück zumindest teilweise einführbar ist, und zumindest eine Testschnittstelle aufweist, welche derart angeordnet ist, dass die zumindest eine Kontaktschnittstelle des zumindest teilweise in die Öffnung eingeführten Kontaktstücks mit der zumindest einen Testschnittstelle elektrisch verbindbar ist.

Weiterhin wird diese Aufgabe durch ein System der eingangs genannten Art dadurch gelöst, dass das Testsystem ein derartiges Testgerät und eine Steuereinheit aufweist, wobei die Steuereinheit mit der zumindest einen Testschnittstelle elektrisch verbindbar ist, wobei die zumindest eine Testschnittstelle mittels der Steuereinheit mit einem Testsignal und/oder einer Reihe von Testsignalen eines Testzyklus beaufschlagbar ist und wobei eine Fehlermeldung erzeugbar ist, falls das Kontaktstück zumindest teilweise in die Öffnung eingeführt ist und falls eine Signaldifferenz zwischen einem jeweiligen von der Testschnittstelle an die Kontaktschnittstelle übertragenen Signal und dem jeweiligen Testsignal größer als ein vorgebbarer Signaldifferenzwert ist.

Schließlich wird diese Aufgabe durch ein Verfahren der eingangs genannten Art durch die folgenden Verfahrensschritte gelöst:
- zumindest teilweises Einführen des Kontaktstücks in die Öffnung,
- Beaufschlagen der zumindest einen Testschnittstelle mit dem Testsignal und/oder der Reihe von Testsignalen des Testzyklus,
- Ermitteln der Signaldifferenz zwischen dem von der Testschnittstelle an die Kontaktschnittstelle übertragenen Signal und dem Testsignal,
- Erzeugen der Fehlermeldung, falls die ermittelte Signaldifferenz größer als der vorgebbare Signaldifferenzwert ist.

Dabei soll der Begriff "elektrisch verbindbar" bzw. "elektrische Verbindung" derart verstanden werden, dass elektrische Signale übertragen werden können, was beispielsweise kabelgebunden oder drahtlos über gängige Funktechnik bzw. auch optisch erreicht werden kann.

Die Öffnung des erfindungsgemäßen Testgeräts ist dabei für ein zugehöriges Kontaktstück einer entsprechenden Messlanze ausgelegt, indem die Abmessungen des Adapterstücks und der Öffnung das zumindest teilweise Einführen des Kontaktstücks durch die Öffnung hindurch in das Adapterstück möglich ist. Weiterhin ist bei einem zumindest teilweise in das Adapterstück eingeführten Kontaktstück die zumindest eine Testschnittstelle dazu ausgelegt, jeweils eine elektrische Verbindung mit der zumindest einen Kontaktschnittstelle zu ermöglichen. Dabei kann auch eine Messlanze mit einer Mehrzahl von Kontaktschnittstellen vorgesehen sein, wobei das Adapterstück des erfindungsgemäßen Testgeräts des entsprechend eine Mehrzahl von Testschnittstellen aufweist und jeweils eine elektrische Verbindung zwischen einer Kontaktschnittstelle und einer Testschnittstelle gewährleistet werden kann.

Bei dem erfindungsgemäßen Testgerät ist von Vorteil, dass das Kontaktstück zumindest teilweise in das Adapterstück eingeführt werden kann. Dies erlaubt zuverlässig und unter Ausschluss möglicher Fehlerquellen, das Testgerät korrekt mit dem Kontaktstück der Messlanze zu verbinden. Insbesondere bei Kontaktstücken bzw. Adapterstücken mit mehreren Kontaktschnittstellen bzw. Testschnittstellen wird dadurch ein automatisiertes Testverfahren ermöglicht, wodurch vermieden werden kann, dass ein Instandhalter sich in die Gefahrenzone begeben muss, um persönlich eine Reinigung durchzuführen. Wenn die zumindest eine Testschnittstelle im Innern des Adapterstücks angeordnet ist, wird dadurch zusätzlich gewährleistet, dass während der Durchführung eines Funktionstests keine weiteren äußeren Umwelteinflüsse den Testvorgang beeinträchtigen können.

Dabei können das Kontaktstück und gegebenenfalls das Adapterstück derart ausgebildet sein, dass ein "Einfädeln" des Kontaktstücks in die Öffnung des Adapterstücks erleichtert wird. Dies kann beispielsweise dadurch erreicht werden, dass das Kontaktstück ein spitzes bzw. konusförmiges Ende, welches in das Adapterstück eingeführt wird, und gegebenenfalls das Adapterstück eine Fase um die Öffnung herum aufweist.

Beispielsweise kann das Adapterstück zumindest eine Aussparungen bzw. Nut aufweisen, welche als Führung dient, wobei das Kontaktstück zumindest einen entsprechenden Steg oder zumindest eine entsprechende Federn aufweist, welche beim zumindest teilweisen Einführen des Kontaktstücks in das Adapterstück in die Führung des Adapterstücks eingreift. Dadurch wird eine besonders zuverlässige elektrische Verbindung der zumindest einen Kontaktschnittstelle mit der zumindest einen Testschnittstelle ermöglicht, weil die jeweiligen Schnittstellen genau und zuverlässig relativ zueinander positioniert werden können. Gegebenenfalls ist weiterhin vorgesehen, dass das Kontaktstück im Adapterstück arretiert wird, beispielsweise mittels einer Schnappverbindung, welche beim zumindest teilweisen Einführen des Kontaktstücks in das Adapterstück einrastet und gegebenenfalls erst nach einem entsprechenden Befehl durch die Steuereinheit gelöst wird, so dass das Kontaktstück wieder aus dem Adapterstück entnommen werden kann.

Weiterhin kann das zumindest teilweise Einführen des Kontaktstücks in die Öffnung durch eine Relativbewegung des Adapterstücks in Bezug auf das Kontaktstück erzielt werden. Insbesondere wird dies dadurch erreicht, dass das Kontaktstück bewegt wird und das Adapterstück ruht bzw. dass das Adapterstück bewegt wird und das Kontaktstück ruht. Zum gleichen Zweck kann alternativ auch eine Bewegung sowohl des Kontaktstücks als auch des Adapterstücks ausgeführt werden.

Der vorgebbare Signaldifferenzwert kann dabei vorab definiert und hinterlegt werden oder beispielsweise mittels eines vorab festgelegten Verfahrens bzw. Rechenmodells bestimmt werden.

Denkbar ist dabei auch, dass ein statistisches Verfahren zum Einsatz kommt. Im Fazit soll der vorgebbare Signaldifferenzwert somit durch vorher bestimmte Kriterien oder Methoden erhältlich sein.

Bei einer vorteilhaften Ausgestaltung der Erfindung weist das Testgerät einen Detektor auf, mittels welchem detektierbar ist, ob das Kontaktstück zumindest teilweise in die Öffnung eingeführt ist.

Dabei kann der Detektor derart angeordnet sein, dass gleichzeitig erfasst wird, ob sich das Kontaktstück relativ zum Adapterstück in der korrekten Lage befindet und somit jeweils eine zuverlässige Verbindung zwischen der zumindest einen Kontaktschnittstelle und der zumindest einen Testschnittstelle gewährleistet sind. Dabei kann der Detektor elektrisch mit der Steuereinheit verbunden sein und das entsprechende Signal an die Steuereinheit übermitteln. Durch den Detektor wird somit wiederum ein automatisierter Testvorgang erleichtert bzw. die Zuverlässigkeit eines solchen Testvorganges erhöht.

Der Detektor kann beispielsweise als optischer Detektor, insbesondere in Form einer Lichtschranke oder einer Kamera, ausgeführt sein oder eine Berührung erfassen.

Bei einer weiteren vorteilhaften Ausgestaltung der Erfindung weist das Testgerät eine Reinigungsvorrichtung auf, mittels welcher die zumindest eine Kontaktschnittstelle reinigbar ist.

Sollte während des Testvorganges festgestellt werden, dass die zumindest eine Kontaktschnittstelle des Kontaktstücks beeinträchtigt ist, ist zu befürchten, dass bei einer Messung von Eigenschaften einer Schmelze mittels der in die Schmelze hineingelassenen Messlanze fehlerhafte Messwerte ermittelt werden. Um die Beeinträchtigung, insbesondere Verschmutzung der zumindest einen Kontaktschnittstelle zu beseitigen, weist das Testgerät eine Reinigungsvorrichtung auf. Dadurch wird wiederum ein automatisierter Testvorgang erleichtert und eine erhöhte Zuverlässigkeit des Testvorganges erreicht. Weiterhin kann selbst im Falle einer erforderlichen Reinigung der zumindest einen Kontaktschnittstelle darauf verzichtet werden, dass ein Instandhalter sich in die Gefahrenzone begeben muss, um persönlich eine Reinigung durchzuführen.

Die Reinigungsvorrichtung kann beispielsweise im Innern des Adapterstücks angeordnet sein, so dass unmittelbar beim zumindest teilweisen Einführen bzw. Herausziehen des Kontaktstücks in das bzw. aus dem Adapterstück eine Reinigung des zumindest einen Kontaktschnittstelle vorgenommen werden kann. Denkbar ist auch, die Reinigungsvorrichtung an der äußeren Oberfläche des Adapterstücks vorzusehen. Bei einer erforderlichen Reinigung der zumindest einen Kontaktschnittstelle wird das Kontaktstück relativ zum Adapterstück zunächst derart bewegt, dass die Reinigungsvorrichtung die zumindest eine Kontaktschnittstelle erreicht und reinigen kann. Anschließend kann das Kontaktstück wiederum zumindest teilweise in das Adapterstück eingeführt werden, um den Testvorgang aufzunehmen.

Gegebenenfalls kann die Reinigungsvorrichtung auch derart gestaltet werden, dass eine Reinigung der zumindest einen Testschnittstelle durchführbar ist. Dies ist insbesondere dann von Vorteil, wenn die zumindest eine Kontaktschnittstelle verschmutzt ist und die Verschmutzung an die zumindest eine Testschnittstelle übergeben wurde.

Insbesondere kann die Reinigungsvorrichtung der Gestalt sein, dass die zumindest eine Kontaktschnittstelle mittels Druckluft oder anderem flüssigen oder gasförmigen Medium abgereinigt wird. Dazu kann die Reinigungsvorrichtung beispielsweise eine Reinigungsdüse aufweisen, welche beispielsweise schwenkbar ausgeführt ist und mittels der Steuereinheit steuerbar ist.

Bei einer weiteren vorteilhaften Ausgestaltung der Erfindung umfasst die Reinigungsvorrichtung einen Motor und eine mittels des Motors bewegbare Reinigungsbürste.

Der Motor kann dabei beispielsweise elektrisch oder mit Druckluft betriebenen werden, je nachdem welche Versorgungsleitungen einfacher dem Adapterstück zuführbar sind. Durch den Motor wird eine Reinigungsbürste angetrieben, welche dazu geeignet ist, die zumindest eine Kontaktschnittstelle von gängigen Verschmutzungen bzw. Belägen zu befreien, ohne die zumindest eine Kontaktschnittstelle dabei zu beschädigen. Dabei kann vorgesehen sein, dass die Reinigungsbürste rotiert und die zumindest eine Kontaktschnittstelle während des Reinigungsvorganges radial außen anzuordnen ist, insbesondere wenn die Reinigungsbürste an der äußeren Oberfläche des Adapterstücks angeordnet ist. Ist die Reinigungsbürste im Innern des Adapterstücks angeordnet, kann wiederum vorgesehen sein, dass die Reinigungsbürste rotiert, dabei jedoch die zumindest eine Kontaktschnittstelle radial innen bezüglich der Reinigungsbürste anzuordnen ist.

Das erfindungsgemäße Testsystem erlaubt einen automatisierten und mannlosen Testvorgang, insbesondere zur Simulation von Sensorsignalen, wie z.B. von Thermospannungen eines Thermoelements, wobei insbesondere erfasst werden soll, ob die zumindest eine Kontaktschnittstelle einen fehlerlosen Messvorgang der Messlanze ermöglicht. Insbesondere ist hierzu eine elektrische Verbindung vorgesehen, welche die Steuereinheit mit der zumindest einen Testschnittstelle elektrisch verbindet, wobei eine weitere elektrische Verbindung vorgesehen sein kann, welche die Steuereinheit mit der Kontaktschnittstelle verbindet. Insbesondere kann die Steuereinheit weitere Aufgaben durchführen, wie beispielsweise die Steuerung des Konverterprozesses oder weiterer Prozessschritte bei der Eisen- oder Stahlherstellung. Alternativ kann auch eine manuelle Messung durchführt werden, welche eine erhöhte Messgenauigkeit erlaubt.

Dabei ist die Steuereinheit dazu ausgelegt, dass Testsignal bzw. die Reihe von Testsignalen eines Testzyklus bereitzustellen, mit welchen die zumindest eine Testschnittstelle beaufschlagt wird. Der Testvorgang sieht zunächst vor, das Kontaktstück zumindest teilweise in die Öffnung einzuführen. Anschließend wird ein Vergleich zwischen einem jeweiligen von der Testschnittstelle an die Kontaktschnittstelle übertragenen Signal und dem jeweiligen Testsignal vorgenommen und eine entsprechende Signaldifferenz erfasst. Hierzu kann die zumindest eine Kontaktschnittstelle mittelbar oder unmittelbar mit der Steuereinheit elektrisch verbunden sein, um den Vergleich zur Ermittlung der Signaldifferenz durchzuführen. Ist die ermittelte Signaldifferenz größer als ein vorgebbarer Signaldifferenzwert, ist eine Fehlermeldung erzeugbar, welche signalisiert, dass die zumindest eine Kontaktschnittstelle nicht in einem ordnungsgemäßen Zustand ist.

Beispielsweise kann eine Mehrzahl von Kontaktschnittstellen vorgesehen sein, mittels welchen während eines Messvorganges verschiedene Messgrößen übermittelt werden können, wie beispielsweise die Temperatur, der Kohlenstoffgehalt oder ähnliche physikalische Größen der Schmelze. Die verschiedenen Kontaktschnittstellen können dann von der Steuereinheit mit einem jeweiligen Testsignal bzw. einer jeweiligen Reihe von Testsignalen eines Testzyklus beaufschlagt werden, wobei jeweils eine Signaldifferenz ermittelt werden kann.

Insbesondere wenn vorgesehen ist, dass das Kontaktstück im Adapterstück arretiert wird, kann die Steuereinheit derart ausgelegt sein, dass sie nach Durchführung eines Testvorganges einen Befehl ausgibt, um die Arretierung zu lösen, so dass das Kontaktstück wieder aus dem Adapterstück entnommen werden kann.

Das Funktionsprinzip kann dabei wie folgt beschrieben werden. Die Steuereinheit startet über die Testschnittstelle genau definierte Messzyklen, wie zum Beispiel die Ausgabe der zu einer gewissen Temperatur gehörigen Thermospannung am Testgerät. Durch den Vergleich der Sollwerte am Adapterstück mit den gemessenen Ist-Werten am Kontaktstück können Rückschlüsse über eine mögliche Abweichung der Messergebnisse getroffen werden.

Durch die vorliegende Erfindung kann nun erstmals ein geschlossener Messkreis realisiert werden, wodurch alle einfließenden Umweltparameter, wie verschmutzte Kontaktstücke, zerstörte bzw. abgebrannte Kontaktschnittstellen, Kabelbruch, erhöhte Übergangswiderstände oder Störungen am Signalkabel, berücksichtigt und automatisiert ausgewertet werden können. Dabei kann der Algorithmus, welcher Soll- und Ist-Kurve vergleicht, in weiterer Folge entsprechende Maßnahmen setzen, wie zum Beispiel das Verschicken einer Information an das Instandhaltungspersonal, wie zum Beispiel "Messung o.k.".

Insgesamt weist die Erfindung ein sehr hohes wirtschaftliches Potenzial auf, da bis dato kein derartiges, automatisiertes System auf dem Markt bekannt ist. Von Vorteil ist weiterhin, dass die Qualität der Flüssigmetallschmelze in einem sehr sensiblen Bereich der Stahlerzeugung gesichert werden kann, dass ein schneller, automatisierter und einfacher Plausibilitätscheck der Messkette ermöglicht wird und dass eine automatische Systemdiagnose mittels einer Messwertsimulation nach jeder Charge möglich ist, was bisher aufgrund der Gefahren meist nur einmal am Tag manuell durchgeführt wurde. Dabei bewirkt die Erfindung eine Erhöhung der Personensicherheit, da für den Testablauf keine Person und somit kein Aufenthalt in Gefahrenbereichen erforderlich ist. Hinzu kommt, dass alle relevanten Parameter des Messkreises überprüft werden können, insbesondere wie oben ausgeführt zum Beispiel eine Verschmutzung des Kontaktstückes, der Zustand der Kabelisolation und dergleichen. Nicht zuletzt ist die Erfindung auch deshalb vorteilhaft, weil der Anschaffungspreis des erfindungsgemäßen Testgeräts bzw. des erfindungsgemäßen Testsystems im Vergleich zu den finanziellen Verlusten durch qualitative Einbußen der Flüssigmetallschmelze sehr gering ausfallen.

Bei einer weiteren vorteilhaften Ausgestaltung der Erfindung weist das Testsystem einen Manipulator auf, mittels welchem das Kontaktstück zumindest teilweise in die Öffnung einführbar ist.

Der Manipulator ist dabei generell als Vorrichtung zu verstehen, welche dazu in der Lage ist, das Kontaktstück zumindest teilweise in die Öffnung einzuführen. Wie oben erläutert, kann dies durch eine Relativbewegung des Adapterstücks in Bezug auf das Kontaktstück erzielt werden.

Insbesondere kann der Manipulator auch dazu verwendet werden, die oben erläuterte Reinigung durchzuführen. Beispielsweise kann vorgesehen sein, dass der Manipulator das Kontaktstück dreht und somit durch Bewegen der zumindest einen Kontaktschnittstelle an einer Bürste entlang die zumindest eine Kontaktschnittstelle reinigt.

Durch den Manipulator wird somit ermöglicht, dass der Testvorgang vollautomatisch durchgeführt wird. Daher wird auch für das zumindest teilweise Einführen des Kontaktstücks in das Adapterstück kein Instandhalter benötigt, welcher für denselben Vorgang die Gefahrenzone betreten müsste. Beispielsweise kann der Manipulator als Roboter ausgeführt sein.

Weist das Testgerät dabei einen Detektor der oben genannten Art auf, kann die Funktion bzw. ordnungsgemäße zumindest teilweise Einführung des Kontaktstücks in das Adapterstück überprüft bzw. verifiziert werden.

Bei einer weiteren vorteilhaften Ausgestaltung der Erfindung umfasst das Testsystem eine Auswerteeinheit, wobei die Auswerteeinheit mit der zumindest einen Kontaktschnittstelle elektrisch verbindbar ist.

Dabei ist die jeweilige Eigenschaft der Schmelze mittels der Auswerteeinheit auf Grundlage des der jeweiligen Auswerteeinheit zugeführten Sensorsignals ermittelbar. Durch die Auswerteeinheit wird somit ein realistischerer Testvorgang ermöglicht, welcher bessere Aussagen darüber treffen kann, ob die zumindest eine Kontaktschnittstelle einen fehlerlosen Messvorgang ermöglicht. Denn der Vergleich zur Bestimmung der Signaldifferenz kann nunmehr auch das von der Testschnittstelle an die Kontaktschnittstelle übertragene Signal berücksichtigen, um die Signaldifferenz zu bestimmen bzw. zu bestimmen, ob die zumindest eine Kontaktschnittstelle in einwandfreiem Zustand ist. Die Berücksichtigung der Auswerteeinheit erlaubt insbesondere nicht-lineare Zusammenhänge zwischen dem charakteristischen Sensorsignal und der jeweiligen Eigenschaft der Schmelze zu berücksichtigen, welche dazu führen können, dass kleine Abweichungen des Sensorsignals zu deutlich unterschiedlichen, ermittelten Eigenschaften der Schmelze führen. Insbesondere kann eine elektrische Verbindung zwischen der Auswerteeinheit und der Steuereinheit vorgesehen sein.

Bei einer weiteren vorteilhaften Ausgestaltung der Erfindung entspricht das Testsignal und/oder die Reihe von Testsignalen des Testzyklus jeweils dem für die jeweilige Eigenschaft der Schmelze charakteristischen Sensorsignal.

Die derartige Ausgestaltung des Testsignals und/oder der Reihe von Testsignalen des Zyklus erlaubt einen zusätzlich realistischen Testvorgang, wodurch das Testverfahren eine noch höhere Zuverlässigkeit erreicht. Insbesondere ist dies von Vorteil, wenn nicht-lineare Zusammenhänge zwischen dem charakteristischen Sensorsignal und der jeweiligen Eigenschaft der Schmelze vorliegen.

Bei einer weiteren vorteilhaften Ausgestaltung der Erfindung ist dabei die Fehlermeldung alternativ oder zusätzlich erzeugbar, falls das Kontaktstück zumindest teilweise in die Öffnung eingeführt ist und falls eine Eigenschaftsdifferenz zwischen jener Eigenschaft der Schmelze, welche dem von der Testschnittstelle an die Kontaktschnittstelle übertragenen Testsignal und/oder der Reihe von Testsignalen des Testzyklus entspricht, und jener Eigenschaft der Schmelze, welche dem jeweiligen charakteristischen Sensorsignal entspricht, größer als ein vorgebbarer Eigenschaftsdifferenzwert ist.

Durch die Bestimmung der Eigenschaftsdifferenz kann ein höchst realistischer Testvorgang durchgeführt werden, da beispielsweise typische Eigenschaften der Schmelze in charakteristische Sensorsignale umgesetzt werden. Die zumindest eine Testschnittstelle wird folglich von der Steuereinheit mit einem entsprechenden Testsignal bzw. einer entsprechenden Reihe von Testsignalen des Testzyklus beaufschlagt. Alternativ oder zusätzlich wird nun ein Vergleich gezogen zwischen der "beaufschlagten" Eigenschaft, wie sie in Form des Testsignals als Input eingegeben wurde, und der "ermittelten" Eigenschaft, wie sie in Form des von der zumindest einen Testschnittstelle an die zumindest eine Kontaktschnittstelle übermittelten Signals als Output ermittelt wird. Dadurch kann zuverlässig verhindert werden, dass bezüglich der Eigenschaften der Schmelze unrealistische Testsignale verwendet werden und somit fehlerhafte Ergebnisse durch den Testvorgang erzielt werden.

Bei einer weiteren vorteilhaften Ausgestaltung der Erfindung, bei welcher das Testgerät zumindest die Reinigungsvorrichtung aufweist, wird die zumindest eine Kontaktschnittstelle nach dem Erzeugen der Fehlermeldung mittels der Reinigungsvorrichtung gereinigt, wobei anschließend das oben genannte Verfahren zur Bestimmung der Signaldifferenz bzw. der Eigenschaftsdifferenz und gegebenenfalls die Erzeugung der Fehlermeldung wiederholt wird.

Die Reinigungsvorrichtung ermöglicht somit, das festgestellte Problem automatisch zu beheben, wodurch wiederum der Aufenthalt eines Instandhalter in der Gefahrenzone eingespart werden kann. Zusätzlich wird dadurch erlaubt, dass das Verfahren unabhängig von der Anwesenheit von Instandhaltern durchgeführt werden kann, da ein manueller Eingriff nicht erforderlich ist.

Insbesondere bei mehreren Kontaktschnittstellen kann es vorkommen, dass eine oder mehrere Kontaktschnittstelle eine unzureichende elektrische Verbindung mit der jeweiligen Testschnittstelle ermöglichen. Für diesen Fall kann vorgesehen werden, dass lediglich jene Kontaktschnittstelle bzw. Kontaktschnittstellen gereinigt wird bzw. werden, welche eine unzureichende elektrische Verbindung mit der jeweiligen Testschnittstelle ermöglicht bzw. ermöglichen.

Bei einer weiteren vorteilhaften Ausgestaltung der Erfindung wird dabei eine Meldung erzeugt, falls der zuvor genannte Verfahrensschritt der Reinigung des Kontaktstücks und der Wiederholung des oben genannten Verfahrens zur Bestimmung der Signaldifferenz bzw. der Eigenschaftsdifferenz und gegebenenfalls die Erzeugung der Fehlermeldung mindestens ein Mal wiederholt wird, wobei die Meldung darauf hinweist, dass ein Austausch des Kontaktstücks erforderlich ist.

Sollte das aufgetretene Problem nicht allein durch einen oder mehrere Reinigungsvorgänge der zumindest einen Kontaktschnittstelle zu beheben sein, könnte dies darauf hinweisen, dass zum Beispiel ein Kabel gebrochen ist oder andere Umstände eingetreten sind, die einen Eingriff eines Instandhalters erforderlich machen. Die Meldung kann daher insbesondere an den zuständigen Instandhalter gesendet werden, um ihn auf die Erfordernis eines manuellen Eingriffs hinzuweisen. Alternativ oder zusätzlich kann die Meldung auch an die Steuereinheit und gegebenenfalls an die Auswerteeinheit übermittelt werden, um zu verhindern, dass fehlerhafte Messungen physikalischer Größen der Schmelze vorgenommen werden.

Bei einer weiteren vorteilhaften Ausgestaltung der Erfindung wird die Fehlermeldung und/oder gegebenenfalls die Meldung, die auf den erforderlichen Austausch des Kontaktstücks hinweist, an ein mit dem Testsystem verbundenes IT-System übermittelt, insbesondere ein Condition-Monitoring-System oder ein Instandhaltungs-Informations-System.

Die Einbettung des Testgeräts und des Testsystems in ein übergeordnetes IT-System vereinfacht besonders die vollständige Automatisierung des erfindungsgemäßen Testvorganges. Somit ist es beispielsweise möglich, das Verfahren per Fernzugriff durchzuführen und zu überwachen sowie gegebenenfalls auf die ermittelten Signaldifferenzen bzw. Eigenschaftsdifferenzen zuzugreifen. Insbesondere die Ausführung des IT-System als Condition-Monitoring-System ist dabei besonders vorteilhaft, da beispielsweise alle Werkzeuge und Maschinen des kompletten Eisen- oder Stahlherstellungsprozess überwacht werden können und Fehler bzw. Fehlfunktionen in einer ganzheitlichen Sicht maschinenübergreifend festgestellt werden können. Dabei erlaubt das Condition-Monitoring-System auch die Bewertung des Zustandes des Messekreises.

Gegebenenfalls kann durch das IT-System ein Instandhaltungsauftrag erzeugt werden, welcher insbesondere an einen zuständigen Instandhalter geschickt wird.

Im Folgenden wird die Erfindung anhand der in den Figuren dargestellten Ausführungsbeispiele näher beschrieben und erläutert. Es zeigen:
- FIG 1: ein erstes Ausführungsbeispiel des erfindungsgemäßen Testgeräts,
- FIG 2: ein Beispiel eines Kontaktstücks,
- FIG 3: ein Beispiel einer Messlanze,
- FIG 4: das erste Ausführungsbeispiel des erfindungsgemäßen Testgeräts mit einem teilweise eingeführten Kontaktstück,
- FIG 5: ein zweites Ausführungsbeispiel des erfindungsgemäßen Testgeräts,
- FIG 6: ein drittes Ausführungsbeispiel des erfindungsgemäßen Testgeräts,
- FIG 7: ein erstes Ausführungsbeispiel des erfindungsgemäßen Testsystems, und
- FIG 8: ein zweites Ausführungsbeispiel des erfindungsgemäßen Testsystems.

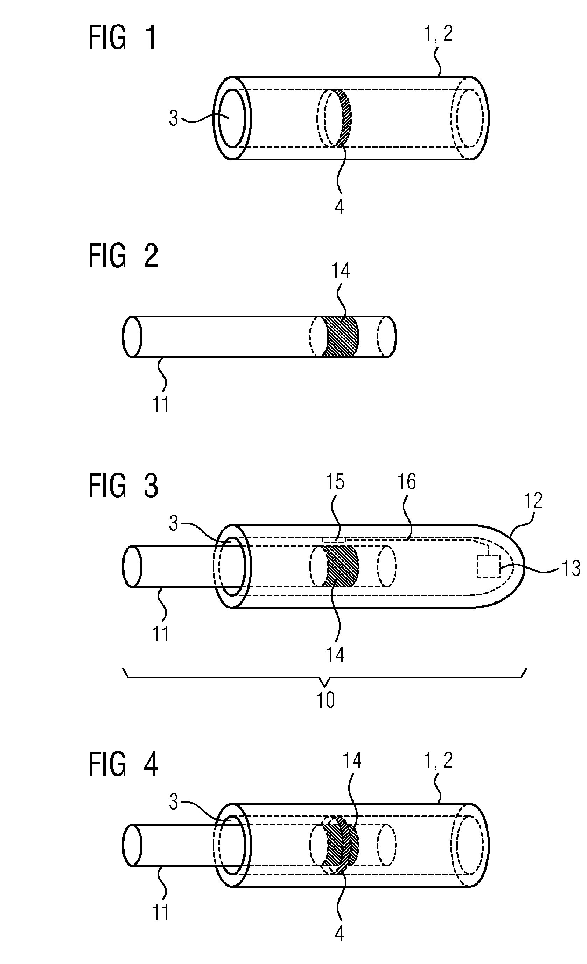

Figur 1 zeigt ein erstes Ausführungsbeispiel des erfindungsgemäßen Testgeräts 1. Das Testgerät 1 weist ein Adapterstück 2 mit einer Öffnung 3 auf. Im Inneren des Testgeräts 1 ist eine Testschnittstelle 4 vorgesehen, welche beispielsweise als zylindermantelförmige oder punktförmige Kontaktstelle ausgebildet sein kann und eine elektrische Verbindung mit einem in Figur 2 näher dargestellten Kontaktstück 11 ermöglicht. Dabei können auch mehrere Testschnittstellen 4 vorgesehen sein, wobei die jeweilige Testschnittstelle 4 auch an der äußeren Oberfläche des Adapterstücks 2 angeordnet sein kann.

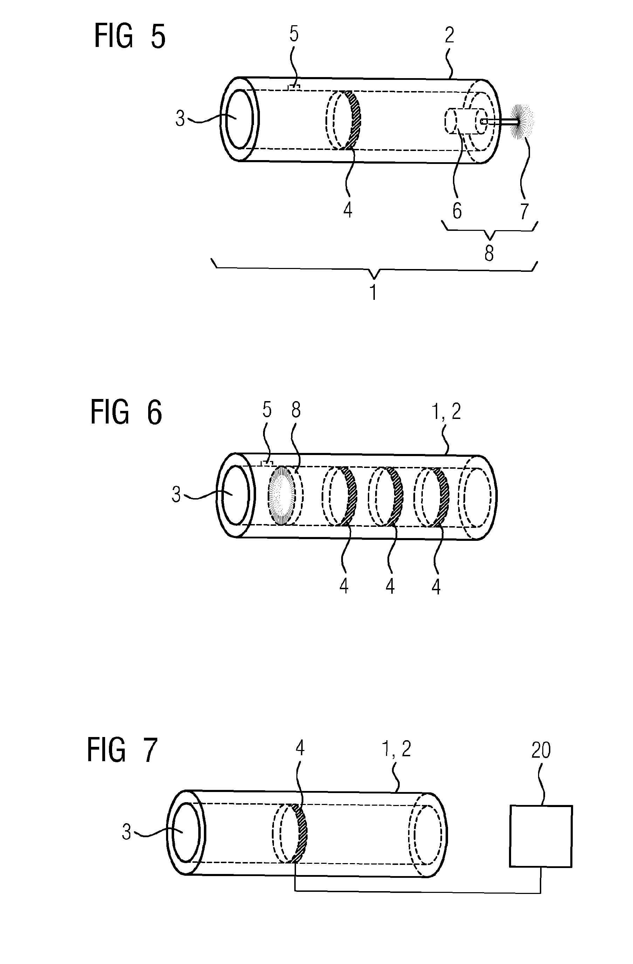

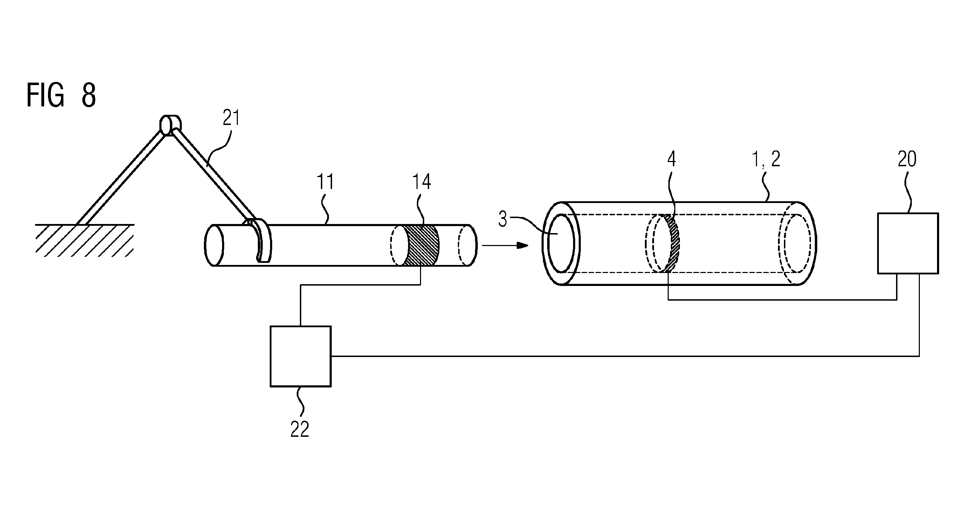

Zur Durchführung des erfindungsgemäßen Verfahrens wird die Testschnittstelle 4 mit einer Steuereinheit 20 verbunden, welche in den Figuren 7 und 8 näher dargestellt ist. Für hier erwähnte und nicht in Figur 1 dargestellte Bezugszeichen, siehe die weiteren Figuren.

Figur 2 zeigt ein Beispiel eines Kontaktstücks 11. Das Kontaktstück 11 weist eine Kontaktschnittstelle 14 auf, wobei die Testschnittstelle 4 eines entsprechend ausgelegten Testgeräts 1 derart angeordnet ist, dass bei ordnungsgemäß in die Öffnung 3 eingeführten Kontaktstück 11 eine elektrische Verbindung zwischen der jeweiligen Testschnittstelle 4 und der jeweiligen Kontaktschnittstelle 14 hergestellt ist. Dabei ist das Kontaktstück 11 Teil einer in Figur 3 dargestellten Messlanze 10 für einen Konverter zur Stahlerzeugung, wobei die Kontaktschnittstelle 14 gegebenenfalls mit einer Auswerteeinheit 22 und/oder mit einer Steuereinheit 20 verbunden wird, welche jeweils in Figur 8 dargestellt ist.

Figur 3 zeigt ein Beispiel einer Messlanze 10. Die Messlanze 10 umfasst dabei das in Figur 2 dargestellte Kontaktstück 11 mit der Kontaktschnittstelle 14. Auf das Kontaktstück 11 ist eine Tauchsonde 12 aufgeschoben, welche zur Erfassung von Messgrößen in flüssiges Eisen bzw. Stahl eingetaucht werden kann. Die Tauchsonde 12 weist dabei einen Sensor 13 auf, welcher mittels einer elektrischen Leitung 16 mit einer Kontaktstelle 15 der Tauchsonde 12 verbunden ist. Während der Messung können vom Sensor 13 erfassten Daten über die elektrische Leitung 16, die Kontaktstelle 15 und die Kontaktschnittstelle 14 an das Kontaktstück 11 übermittelt werden. Die Daten können anschließend zur Auswertung an weitere Einrichtungen übermittelt werden.

Figur 4 zeigt das erste Ausführungsbeispiel des erfindungsgemäßen Testgeräts 1 mit einem teilweise eingeführten Kontaktstück 11. Dabei ist dargestellt, dass das Kontaktstück 11 ordnungsgemäß in das Testgerät 1 eingeführt ist, so dass die Kontaktschnittstelle 14 in elektrischem Kontakt mit der Testschnittstelle 4 ist. Dies erlaubt die Durchführung der Verfahrensschritte des erfindungsgemäßen Testvorganges.

Figur 5 zeigt ein zweites Ausführungsbeispiel des erfindungsgemäßen Testgeräts 1. Im Unterschied zum ersten Ausführungsbeispiel weist das zweite Ausführungsbeispiel des erfindungsgemäßen Testgeräts 1 zusätzlich einen Detektor 5 auf, der erfasst, ob sich das passende Kontaktstück 11 in der Öffnung 3 befindet bzw. eingeführt wird. Weiterhin weist das Testgerät 1 eine Reinigungsvorrichtung 8 auf, welche einen Motor 6 und eine mittels des Motors 6 bewegbare Reinigungsbürste 7 aufweist. Mittels der Reinigungsvorrichtung kann die zumindest eine Kontaktschnittstelle 14 des Kontaktstücks 11 gereinigt werden. Hierzu ist beispielsweise vorgesehen, dass der Manipulator 21 das Kontaktstück 11 dreht und somit durch Bewegen der Kontaktschnittstelle 14 an der Bürste 7 entlang die Kontaktschnittstelle 14 reinigt.

Figur 6 zeigt ein drittes Ausführungsbeispiel des erfindungsgemäßen Testgeräts 1. Im Unterschied zum zweiten Ausführungsbeispiel weist das dritte Ausführungsbeispiel des Testgeräts 1 nunmehr drei Testschnittstellen 4 auf, wobei ein passendes Kontaktstück 11 ebenfalls drei entsprechend angeordnete Kontaktschnittstellen 14 aufweisen würde. Weiterhin ist die Reinigungsvorrichtung 8 nunmehr im Innern des Adapterstücks 2 angeordnet, so dass die jeweilige Kontaktschnittstelle 14 beim Einführen des Kontaktstücks 11 in die Öffnung 3 des Adapterstücks 2 gereinigt werden kann.

Figur 7 zeigt ein erstes Ausführungsbeispiel des erfindungsgemäßen Testsystems. Das Testsystem weist ein Testgerät 1 auf, welches in den weiteren Figuren dargestellt und diskutiert wird. Das Testsystem umfasst weiterhin eine Steuereinheit 20, welche mit der Testschnittstelle 4 des Adapterstücks 2 elektrisch verbunden ist, so dass Testsignale von der Steuereinheit 20 an die Testschnittstelle 4 übermittelt werden können. Bei einem in die Öffnung 3 eingeführten Kontaktstück 11 werden die Testsignale über die Testschnittstelle 4 und die Kontaktschnittstelle 14 an das Kontaktstück 11 übermittelt, wobei die übermittelten Testsignale anschließend für eine Ermittlung einer Signaldifferenz bzw. einer Eigenschaftsdifferenz verwendet werden können.

Figur 8 zeigt ein zweites Ausführungsbeispiel des erfindungsgemäßen Testsystems. Im Unterschied zum ersten Ausführungsbeispiel weist das zweite Ausführungsbeispiel des Testsystems zusätzlich eine Auswerteeinheit 22 auf, welche zum einen mit der Kontaktschnittstelle 14 des Kontaktstücks 11 und zum anderen mit der Steuereinheit 20 elektrisch verbunden ist. Die Auswerteeinheit 22 kann dabei den von der Kontaktschnittstelle 14 erhaltenen Signalen eine physikalische Eigenschaft einer Schmelze zuordnen, so dass durch dieses Testsystem die Ermittlung einer Eigenschaftsdifferenz ermöglicht wird.

Weiterhin weist das Testsystem einen Manipulator 21 auf, mittels welchem das Kontaktstück 11 in die Öffnung 3 des Adapterstücks 2 eingeführt und wieder herausgeholt werden kann, um einen vollständig automatisierten Testvorgang zu ermöglichen. Dabei ist der Manipulator 21 generell derart ausgestaltet, dass das Adapterstück 2 relativ zum Kontaktstück 11 bewegt wird.

Die in den Figuren dargestellten elektrischen Verbindungen dienen der Signalübermittlung und können alternativ drahtlos oder optisch ausgeführt werden.

Zusammenfassend betrifft die Erfindung ein Testgerät für eine Messlanze zur Durchführung von Messungen bei der Erzeugung und Verarbeitung von Flüssigmetallschmelzen, wobei die Messlanze ein Kontaktstück und eine mechanisch lösbar am Kontaktstück befestigbare Tauchsonde aufweist, wobei die Tauchsonde in die Schmelze eintauchbar ist und zumindest einen Sensor zur Ausgabe eines für eine jeweilige Eigenschaft der Schmelze charakteristischen Sensorsignals aufweist, wobei das Kontaktstück zumindest eine Kontaktschnittstelle aufweist, mittels welcher der zumindest eine Sensor mit dem Kontaktstück elektrisch verbindbar ist. Weiterhin betrifft die Erfindung ein Testsystem mit einem derartigen Testgerät und einer Steuereinheit sowie ein Verfahren zum Betrieb eines derartigen Testsystems. Um einen verbesserten Test bezüglich der Funktion eines Kontaktstücks einer Messlanze bereitzustellen, wird vorgeschlagen, dass das Testgerät ein Adapterstück mit einer Öffnung, in welche das Kontaktstück zumindest teilweise einführbar ist, und zumindest eine Testschnittstelle aufweist, welche derart angeordnet ist, dass die zumindest eine Kontaktschnittstelle des zumindest teilweise in die Öffnung eingeführten Kontaktstücks mit der zumindest einen Testschnittstelle elektrisch verbindbar ist.

## Patentansprüche

1. Testgerät (1) für eine Messlanze (10) zur Durchführung von Messungen bei der Erzeugung und Verarbeitung von Flüssigmetallschmelzen,
wobei die Messlanze (10) ein Kontaktstück (11) und eine mechanisch lösbar am Kontaktstück (11) befestigbare Tauchsonde (12) aufweist,
wobei die Tauchsonde (12) in die Schmelze eintauchbar ist und zumindest einen Sensor (13) zur Ausgabe eines für eine jeweilige Eigenschaft der Schmelze charakteristischen Sensorsignals aufweist,
wobei das Kontaktstück (11) zumindest eine Kontaktschnittstelle (14) aufweist, mittels welcher der zumindest eine Sensor (13) mit dem Kontaktstück (11) elektrisch verbindbar ist, umfassend
- ein Adapterstück (2) mit einer Öffnung (3), in welche das Kontaktstück (11) zumindest teilweise einführbar ist,
- zumindest eine Testschnittstelle (4), welche derart angeordnet ist, dass die zumindest eine Kontaktschnittstelle (14) des zumindest teilweise in die Öffnung (3) eingeführten Kontaktstücks (11) mit der zumindest einen Testschnittstelle (4) elektrisch verbindbar ist.

2. Testgerät (1) nach Anspruch 1,
wobei das Testgerät (1) einen Detektor (5) aufweist, mittels welchem detektierbar ist, ob das Kontaktstück (11) zumindest teilweise in die Öffnung (3) eingeführt ist.

3. Testgerät (1) nach einem der vorhergehenden Ansprüche, wobei das Testgerät (1) eine Reinigungsvorrichtung (8) aufweist, mittels welcher die zumindest eine Kontaktschnittstelle (14) reinigbar ist.

4. Testgerät (1) nach Anspruch 3,
wobei die Reinigungsvorrichtung (14) einen Motor (6) und eine mittels des Motors (6) bewegbare Reinigungsbürste (7).

5. Testsystem für eine Messlanze (10) zur Durchführung von Messungen bei der Erzeugung und Verarbeitung von Flüssigmetallschmelzen,
wobei die Messlanze (14) ein Kontaktstück (11) und eine mechanisch lösbar am Kontaktstück (11) befestigbare Tauchsonde (12) aufweist,
wobei die Tauchsonde (12) in die Schmelze eintauchbar ist und zumindest einen Sensor (13) zur Ausgabe eines für eine jeweilige Eigenschaft der Schmelze charakteristischen Sensorsignals aufweist,
wobei das Kontaktstück (11) zumindest eine Kontaktschnittstelle (14) aufweist, mittels welcher der zumindest eine Sensor (13) mit dem Kontaktstück (11) elektrisch verbindbar ist, umfassend
- ein Testgerät (1) nach einem der vorhergehenden Ansprüche,
- eine Steuereinheit (20),
wobei die Steuereinheit (20) mit der zumindest einen Testschnittstelle (4) elektrisch verbindbar ist,
wobei die zumindest eine Testschnittstelle (4) mittels der
Steuereinheit (20) mit einem Testsignal und/oder einer Reihe von Testsignalen eines Testzyklus beaufschlagbar ist, wobei eine Fehlermeldung erzeugbar ist, falls das Kontaktstück (11) zumindest teilweise in die Öffnung (3) eingeführt ist und falls eine Signaldifferenz zwischen einem jeweiligen von der Testschnittstelle (4) an die Kontaktschnittstelle (14) übertragenen Signal und dem jeweiligen Testsignal größer als ein vorgebbarer Signaldifferenzwert ist.

6. Testsystem nach Anspruch 5,
wobei das Testsystem zumindest einen Manipulator (21) aufweist, mittels welchem das Kontaktstück (11) zumindest teilweise in die Öffnung (3) einführbar ist.

7. Testsystem nach Anspruch 5 oder 6,
umfassend eine Auswerteeinheit (22),
wobei die Auswerteeinheit (22) mit der zumindest einen Kontaktschnittstelle (14) elektrisch verbindbar ist.

8. Testsystem nach einem der Ansprüche 5-7,
wobei das Testsignal und/oder die Reihe von Testsignalen des Testzyklus jeweils dem für die jeweilige Eigenschaft der Schmelze charakteristischen Sensorsignal entspricht.

9. Testsystem nach Anspruch 8,
wobei die Fehlermeldung alternativ oder zusätzlich erzeugbar ist, falls das Kontaktstück (11) zumindest teilweise in die Öffnung (3) eingeführt ist und falls eine Eigenschaftsdifferenz zwischen jener Eigenschaft der Schmelze, welche dem von der Testschnittstelle (4) an die Kontaktschnittstelle (14) übertragenen Testsignal und/oder der Reihe von Testsignalen des Testzyklus entspricht, und jener Eigenschaft der Schmelze, welche dem jeweiligen charakteristischen Sensorsignal entspricht, größer als ein vorgebbarer Eigenschaftsdifferenzwert ist.

10. Verfahren zum Betrieb eines Testsystems nach einem der Ansprüche 5-9 mit den folgenden Verfahrensschritten:
- zumindest teilweises Einführen des Kontaktstücks (11) in die Öffnung (3),
- Beaufschlagen der zumindest einen Testschnittstelle (4) mit dem Testsignal und/oder der Reihe von Testsignalen des Testzyklus,
- Ermitteln der Signaldifferenz zwischen dem von der Testschnittstelle (4) an die Kontaktschnittstelle (14) übertragenen Signal und dem Testsignal,
- Erzeugen der Fehlermeldung, falls die ermittelte Signaldifferenz größer als der vorgebbare Signaldifferenzwert ist.

11. Verfahren zum Betrieb eines Testsystems nach Anspruch 9 mit den folgenden Verfahrensschritten:
- zumindest teilweises Einführen des Kontaktstücks (11) in die Öffnung (3),
- Beaufschlagen der zumindest einen Testschnittstelle (4) mit dem Testsignal und/oder der Reihe von Testsignalen des Testzyklus,
- Ermitteln der Eigenschaftsdifferenz zwischen jener Eigenschaft der Schmelze, welche dem von der Testschnittstelle (4) an die Kontaktschnittstelle (14) übertragenen Signal entspricht, und jener Eigenschaft der Schmelze, welche dem Testsignal entspricht,
- Erzeugen der Fehlermeldung, falls die ermittelte Eigenschaftsdifferenz größer als der vorgebbare Eigenschaftsdifferenzwert ist.

12. Verfahren nach Anspruch 10 oder 11,
wobei das Kontaktstück (11) mittels eines Manipulators (21) zumindest teilweise in die Öffnung (3) eingeführt wird.

13. Verfahren nach einem der Ansprüche 10-12 mit einem Testgerät nach Anspruch 3 oder 4,
wobei nach dem Erzeugen der Fehlermeldung die zumindest eine Kontaktschnittstelle (14) mittels der Reinigungsvorrichtung (8) gereinigt wird,
wobei das Verfahren nach Anspruch 9 oder 10 wiederholt wird.

14. Verfahren nach Anspruch 13,
wobei eine Meldung erzeugt wird, falls das Verfahren nach 11 mindestens ein Mal wiederholt wird,
wobei die Meldung darauf hinweist, dass ein Austausch des Kontaktstücks (11) erforderlich ist.

15. Verfahren nach einem der Ansprüche 9-14,
wobei die Fehlermeldung und/oder gegebenenfalls die Meldung, die auf den erforderlichen Austausch des Kontaktstücks hinweist, an ein mit dem Testsystem verbundenes IT-System, insbesondere ein Condition-Monitoring-System oder ein Instandhaltungs-Informations-System, übermittelt wird.
